(19) Europäisches Patentamt
European Patent Office
Office européen des brevets

(11) Veröffentlichungsnummer: 0 039 482 B1

(12) EUROPÄISCHE PATENTSCHRIFT

(45) Veröffentlichungstag der Patentschrift: 27.03.85

(51) Int. Cl.⁴: C 07 D 491/06, C 09 B 5/62 // (C07D491/06, 311:00, 221:00)

(21) Anmeldenummer: 81103257.2

(22) Anmeldetag: 30.04.81

(54) **Perylen-3,4,9,10-tetracarbonsäuremonoanhydridmonoimide, Verfahren zur Herstellung solcher Verbindungen und ihre Verwendung.**

(30) Priorität: 05.05.80 DE 3017185

(43) Veröffentlichungstag der Anmeldung: 11.11.81 Patentblatt 81/45

(45) Bekanntmachung des Hinweises auf die Patenterteilung: 27.03.85 Patentblatt 85/13

(84) Benannte Vertragsstaaten: CH DE FR GB LI

(56) Entgegenhaltungen:
DE - C - 411 217

NIPPON KAGAKU KAISHI, Nr. 4, 1979 Y. NAGAO et al. "Reactions of 3,4:9,10-Perylenetetracarboxylic Dianhydride with Alkylamines. III. Synthesis and Reactions of perylenecarboxylic Acid Derivatives" Seiten 528 bis 534
KOGYO KAGAKU ZASSHI, Band 74, Nr. 10 1971 Seiten 2500 bis 2502
Chemical Abstracts Band 91, Nr. 4 23. Juli 1979 Columbus, Ohio, USA Y. NAGAO et al. "Synthesis of unsymmetrical perylenebis(dicarboximide) derivatives" Seite 93, Spalte 1, Abstract Nr. 22404r

(73) Patentinhaber: **HOECHST AKTIENGESELLSCHAFT, Postfach 80 03 20, D-6230 Frankfurt am Main 80 (DE)**

(72) Erfinder: **Spietschka, Ernst, Dr., Kirchweg 3, D-6270 Idstein/Taunus (DE)**
Erfinder: **Tröster, Helmut, Dr., Am Erdbeerstein 44, D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 039 482 B1

BUNDESDRUCKEREI BERLIN

**Beschreibung**

Die Erfindung betrifft Perylen-3,4,9,10-tetracarbonsäuremonoanhydridmonoimide der Formel 1

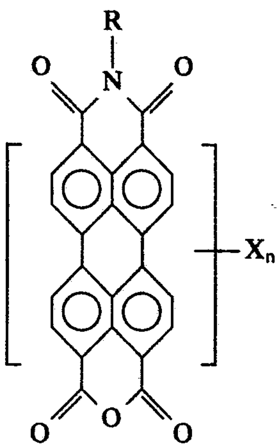

(1)

in der

R Wasserstoff,
Hydroxy,
Amino,
Cycloalkyl mit 4 bis 8 C-Atomen,
Alkyl mit 1 bis 8 C-Atomen, das substituiert sein kann durch
- Cycloalkyl mit 4 bis 8 C-Atomen,
- Phenyl, das seinerseits substituiert sein kann durch
  - Halogen,
  - niederes Alkyl,
  - niederes Alkoxy oder
  - niederes Alkoxycarbonyl,
- Cyan,
- Hydroxy,
- Carbamoyl,
- Acyl,
- niederes Dialkylamino,
- Morpholyl,
- Piperidyl oder
- Alkoxy mit 1 bis 8 C-Atomen, das seinerseits substituiert sein kann durch
  - Hydroxy,
  - niederes Alkoxy,
  - niederes Hydroxyalkyl,
  - Cyan,
  - Cycloalkyl mit 4 bis 8 C-Atomen oder
  - Phenyl,

X Chlor oder Brom und
n eine Zahl von 0 bis 4
bedeuten, wobei R nicht die Gruppe

$$-(CH_2)_y-H$$

in der y 0 bis 4 ist, bedeutet, wenn n Null ist.

Besonders bevorzugt sind diejenigen Verbindungen, in denen $n = 0$ bedeutet.

Weiterhin betrifft die Erfindung ein Verfahren zur Herstellung von Perylen-3,4,9,10-tetracarbonsäuremonoanhydridmonoimiden der Formel 1, in der R für Wasserstoff, Amino, Hydroxy oder eine gegebenenfalls substituierte Alkylgruppe steht, X Chlor oder Brom und n eine Zahl von 0–4 bedeutet.

Die Herstellung von Perylen-3,4,9,10-tetracarbonsäuremonoanhydridmonoimid durch partielle Verseifung des entsprechenden Diimids mit Schwefelsäure ist bekannt (DE-PS 411 217). Das Verfahren liefert jedoch nur eine Ausbeute von 55% (Nagao et al., Kogyo Kagaku Zasshi 1971, 74 (12), 2500–2).

In Nippon Kagaku Kaishi, 1979 (4), S. 528–34 wird die Reaktion von Perylen-3,4,9,10-tetracarbonsäuredianhydrid mit Methyl-, Ethyl-, Propyl- und Butylamin, die über die entsprechenden Monoimide zu

den Diimiden führt, untersucht. Die intermediär gebildeten entsprechenden Monoimide werden im Reaktionsgemisch spektralfotometrisch bestimmt, wobei Maximalausbeuten von 62—84% angegeben werden (Abschnitt 2.3 und 2.4, S. 529 und Tab. 3, S. 530). Die tatsächlich erzielten Ausbeuten an dem aus der Reaktionsmischung isolierten Monomethyl-, Monoethyl-, Monopropyl- und Monobutylamid betrugen jedoch nur 24—46% (Abschnitt 2.2 und Tab. 1, S. 529). Neben den unbefriedigenden Ausbeuten hat diese Verfahrensweise den Nachteil, daß sie eine ständige analytische Überprüfung des Reaktionsablaufs erfordert.

Es wurde nun gefunden, daß man Perylen-3,4,9,10-tetracarbonsäuremonoanhydridmonoimide der Formel 1 erhalten kann, wenn man Perylen-3,4,9,10-tetracarbonsäuremonoanhydridmonoalkalisalze der Formel 2

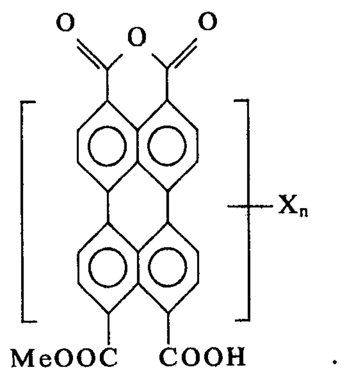

(2)

in der Me für ein Natrium- oder Kaliumatom steht und X und n die vorstehend genannte Bedeutung haben, mit Verbindungen der Formel 3

$$R—NH_2$$

worin R Wasserstoff, Amino, Hydroxy, oder eine gegebenenfalls substituierte Alkylgruppe bedeutet, bei 0°—130°C, vorzugsweise bei 0°—95°C umsetzt.

Das neue Verfahren liefert in einfacher Weise, ohne daß es einer besonderen Kontrolle des Reaktionsverlaufes bedarf, die entsprechenden Perylen-3,4,9,10-tetracarbonsäuremonoanhydridmonoimide in hohen, teilweise nahezu quantitativen Ausbeuten.

Im folgenden werden bevorzugte Ausgestaltungen der Erfindung näher erläutert:

Die Ausgangsverbindungen der Formel 2 können nach dem in der EP-A-35 218 (81 101 315.0) beschriebenen Verfahren hergestellt werden. Hierbei wird eine wäßrige Lösung oder Suspension eines Tetraalkali- oder Tetraammoniumsalzes der entsprechenden Perylen-3,4,9,10-tetracarbonsäure bei etwa 20—100°C, vorzugsweise 70—95°C, mit drei Äquivalenten Säure, zweckmäßig starker Mineralsäure wie Salz-, Schwefel-, Salpeter- oder Phosphorsäure, versetzt. Wird ein Ammoniumsalz eingesetzt, so muß mindestens ein Äquivalent Natrium- oder Kaliumionen zugegen sein. Dabei ist eine Zwischenisolierung dieser Verbindungen nicht erforderlich. Sie können auch so, wie sie bei der Synthese im Reaktionsgemisch anfallen, mit den Verbindungen der Formel 3 umgesetzt werden.

Als Ausgangsstoffe der Formel 2 sind besonders diejenigen bevorzugt, bei denen in der Formel n = 0 und Me Kalium bedeutet.

Ausgangsstoffe der Formel 3 sind beispielsweise

Ammoniak, Hydrazin, Hydroxylamin, Methyl-, Ethyl-, Propyl-, Butyl-, Octylamin, Benzylamin, Ethanol-, Isopropanol-, Hydroxypropylamin, Methoxypropyl-, Butoxypropyl-, Butoxyethoxypropyl-, Octyloxypropyl-, 2-Ethylhexoxypropyl-, 3-Morpholinopropylamin, Cyclohexylamin, 2-Cyanethylamin, 2-Chlor- oder Bromethylamin, Carbamoylethylamin, Cyclohexoxypropyl-, Benzyloxypropylamin.

Pro Mol Ausgangsstoff der Formel 2 werden mindestens 2 Mol, zweckmäßig 3 Mol oder auch ein größerer Überschuß der Verbindung 3 eingesetzt. Die Kondensation wird in Wasser bei Temperaturen von 0°—130°C, vorzugsweise bei 0°—95°C durchgeführt. Dabei verfährt man zweckmäßig so, daß man in ein Amin-Wassergemisch das Monoanhydridmonoalkalisalz einträgt oder zur wäßrigen Suspension des Monoanhydridmonoalkalisalzes das Amin der Formel 3 zugibt.

Die Vereinigung der Reaktionskomponenten kann bei 0°—95°C erfolgen. Zweckmäßig wird man die Reaktionsmischung bei niederen Temperaturen von etwa 0°—30°C herstellen und anschließend die Kondensation bei höheren Temperaturen von etwa 70°—95°C zu Ende führen. Bei 90°—95°C ist die Umsetzung zum Monoimid im allgemeinen nach 1—2 Stunden beendet.

Aus dem Reaktionsgemisch wird dann durch Ansäuern, beispielsweise mit Mineralsäure, zweckmäßig bei höherer Temperatur von etwa 70°–95°C, das Reaktionsprodukt erhalten. Es wird in üblicher Weise, beispielsweise durch Filtration, isoliert. Gegebenenfalls kann es noch von geringen Mengen an mitentstandenem Diimid sowie von Perylentetracarbonsäuredianhydrid beispielsweise gemäß den in Nippon Kagaku Kaishi a. a. O., Abschn. 2.2., S. 529 gemachten Angaben befreit werden, indem man das alkaliunlösliche Diimid durch eine alkalische Klärfiltration entfernt und das Perylentetracarbonsäuredianhydrid über sein leicht lösliches Tetrakaliumsalz von dem häufig schwer löslichen Dikaliumsalz des Endproduktes abtrennt.

Die erhaltenen Verfahrensprodukte sind zum großen Teil neu. Sie stellen wertvolle Ausgangsprodukte zur Herstellung von Farbmitteln, insbesondere von asymmetrisch N-substituierten Perylen-3,4,9,10-tetracarbonsäurediimiden, dar.

In den folgenden Beispielen beziehen sich Prozentangaben auf das Gewicht, sofern nichts anderes angegeben ist.

Beispiel 1

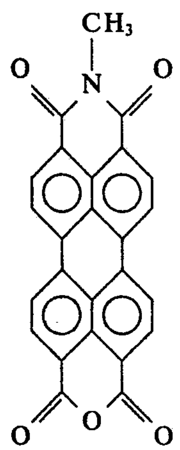

Zu einer Suspension von 22,4 g des Monokaliumsalzes des Perylen-3,4,9,10-tetracarbonsäuremonoanhydrids in 250 ml Wasser werden bei 0–5°C, 10,3 g Methylaminlösung (45,4%ig) zugegossen und 12 Stunden bei 0–5°C gerührt. Anschließend gibt man 27,0 g 50%ige Kalilauge zu und hält die Temperatur der Mischung 1 Stunde bei 90°C. Das Reaktionsprodukt wird bei 20–25°C abgesaugt und zur Entfernung von Spuren des Tetrakaliumsalzes der Perylentetracarbonsäure mit 5%iger Kalilauge bis zum farblosen Filtratablauf gewaschen. Der Filterrückstand wird in 300 ml Wasser bei 90–95°C gelöst und heiß von Spuren Perylentetracarbonsäurediimid abfiltriert. Aus dem Filtrat wird das Perylen-3,4,9,10-tetracarbonsäure-monoanhydrid-monomethylimid durch Ansäuern mit conc. Salzsäure ausgefällt, abgesaugt, chloridfrei gewaschen und getrocknet.

Ausbeute: 19,4 g (95,4% d. Th.).

| | | | | |
|---|---|---|---|---|
| Analyse: | ber. | C 74,1% | gef. | C 73,7% |
| | | H 2,7% | | H 2,8% |
| | | N 3,5% | | N 3,5% |

Beispiel 2

124 g Methylamin (45,4%ig) werden bei 20–25°C zu einer Suspension des Monokaliumsalzes des Perylentetracarbonsäuremonoanhydrids gegeben, die gemäß Beispiel 3 der Patentanmeldung 81 101 315.0 aus 196 g Perylentetracarbonsäuredianhydrid (ohne Zwischenisolierung des Monokaliumsalzes) erhalten wurde. Anschließend wird auf 90–95°C erwärmt. Nach 1 Stunde läßt man 160 g 50%ige Kalilauge zulaufen und rührt noch 1 Stunde bei gleicher Temperatur nach.

Das auskristallisierte rote Dikaliumsalz des Semimethylimids wird bei Raumtemperatur abgesaugt, mit einer wäßrigen Lösung aus 3% Kaliumchlorid und 1% Kaliumhydroxid bis zum farblosen Filtratablauf gewaschen und wie in Beispiel 1 angegeben zum identischen Endprodukt aufgearbeitet.

Ausbeute: 191,7 g (94,7% d. Th. bezogen auf Perylentetracarbonsäuredianhydrid).

Beispiel 3

Arbeitet man wie in Beispiel 1 angegeben, verwendet jedoch anstelle des Monokaliumsalzes das Mononatriumsalz (Na-Gehalt 3,5% = 65,8% Mono-Natriumsalz) so erhält man 13,0 g (64,2%) identisches Endprodukt.

Beispiel 4

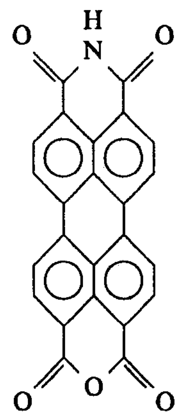

In 280 g wäßrige 3%ige Ammoniaklösung werden bei 0—5°C 22,4 g Monokaliumsalz des Perylentetracarbonsäuremonoanhydrids eingetragen und es wird 4 Stunden ohne weitere Kühlung nachgerührt. Anschließend erwärmt man auf 90—95°C und rührt 2 Stunden bei dieser Temperatur nach. Nach Zugabe einer Lösung von 15 g Kaliumcarbonat (wasserfrei) in 50 ml Wasser wird eine weitere Stunde bei 90°C nachgerührt. Bei 20—25°C saugt man ab und wäscht zur Entfernung einer geringen Menge des Tetrakaliumsalzes der Perylentetracarbonsäure mit 2%iger Kaliumcarbonatlösung bis zum farblosen Filtratablauf.

Der Rückstand wird in 1300 g, 3,5%iger Kaliumhydroxidlösung bei 95°C gelöst und durch Filtration eine Spur Perylentetracarbonsäurediimid abgetrennt. Aus dem heißen Filtrat wird mit Salzsäue (31%ig) das Reaktionsprodukt ausgefällt und wie üblich isoliert. Man erhält 17,9 g (91,6%) Perylentetracarbonsäuremonoanhydridmonoimid.

Analyse: ber. N 3,5%, gef. N 3,5%.

Beispiel 5

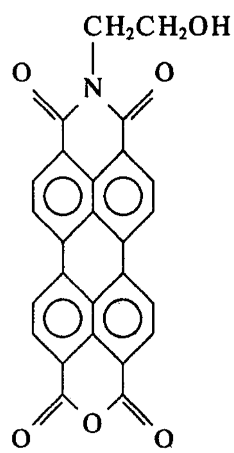

In eine wäßrige Lösung von 13,5 g Ethanolamin in 250 ml Wasser werden 22,4 g Monokaliumsalz des Perylentetracarbonsäuremonoanhydrids eingetragen, 2 Stunden bei 20—30°C und 2 Stunden bei 90—95°C gerührt. Anschließend wird die dunkelrote Lösung heiß geklärt, durch Ansäuern das Reaktionsprodukt ausgefällt und in bekannter Weise isoliert. Zur Entfernung einer geringen Menge Perylentetracarbonsäure wird es in 200 ml 5%iger Kalilauge auf 90°C erwärmt und durch Zusatz von 20 g Kaliumchlorid das Di-Kaliumsalz ausgesalzen. Das bei Raumtemperatur abgesaugte Produkt wird mit

einer wäßrigen Lösung aus 3% Kaliumchlorid und 1% Kaliumhydroxid gewaschen, der Rückstand in 500 ml Wasser gelöst und in üblicher Weise sauer gefällt und isoliert.

Ausbeute: 20,0 g (91,9%).

| | | | | |
|---|---|---|---|---|
| Analyse: | ber. | C 71,7% | gef. | C 71,0% |
| | | H 3,0% | | H 3,0% |
| | | N 3,2% | | N 3,2% |

Beispiel 6

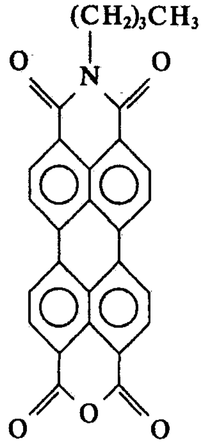

22,4 g Monokaliumsalz des Perylentetracarbonsäuremonoanhydrids werden bei Raumtemperatur in eine Lösung von 14,6 g Butylamin in 250 ml Wasser eingetragen und 5 Stunden bei 20—25°C und 1 Stunde bei 90°C gerührt. Anschließend wird das Reaktionsprodukt sauer gefällt und isoliert. Zur Abtrennung von geringen Mengen Diimid und Perylentetracarbonsäure löst man die Substanz in 350 ml 5%iger Kalilauge bei 90—95°C und fällt durch Zusatz von 30 g Kaliumchlorid das Dikaliumsalz. Es wird bei Raumtemperatur abgesaugt und mit einer wäßrigen Lösung von 14% Kaliumchlorid und 1% Kaliumhydroxid gewaschen. Der in Wasser bei Siedetemperatur gelöste Rückstand wird von Spuren Diimid geklärt. Durch Ansäuern des Filtrats und übliche Isolierung werden 19,4 g (86,8%) Perylentetracarbonsäuremonoanhydridmonobutylimid erhalten.

Analyse: ber. N 3,1%, gef. N. 3,1%.

Beispiel 7

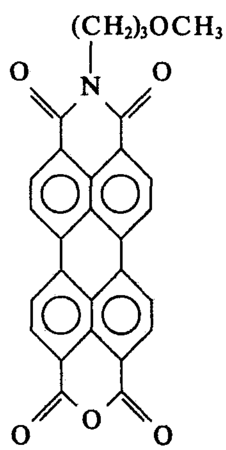

Wie in Beispiel 6 angegeben werden 22,4 g Monokaliumsalz des Perylentetracarbonsäuremonoanhydrids mit 19,6 g 3-Methoxypropylamin umgesetzt. Vor der sauren Fällung wird nach Verdünnen mit 500 ml Wasser von einer geringen Menge Diimid geklärt. Man erhält 22,0 g (95,0%) Reaktionsprodukt.

Analyse: ber. N 3,0%, gef. N 2,7%.

Beispiel 8

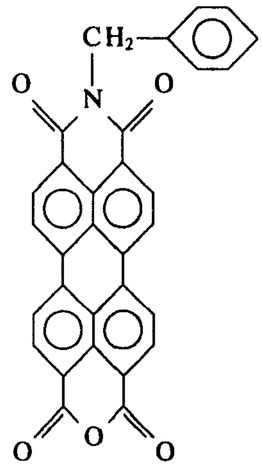

44,8 g Monokaliumsalz des Perylentetracarbonsäuremonoanhydrids trägt man in eine eiskalte Lösung von 47 g Benzylamin in 500 ml Wasser ein, erwärmt langsam auf 90—95°C, gibt nach 2 Stunden 60 g 50%ige Kalilauge zu und salzt mit 25 g Kaliumchlorid das Dikaliumsalz aus. Nach Aufarbeitung gemäß Beispiel 7 erhält man das Perylentetracarbonsäuremonoanhydridmonobenzylimid in einer Ausbeute von 44,0 g (91,5%).

Analyse: ber. N 2,9%, gef. N 2,6%.

Beispiel 9

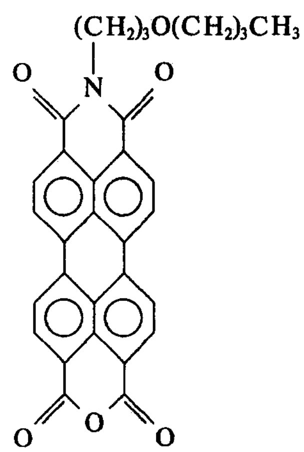

Man verfährt gemäß Beispiel 7, wobei anstelle von 3-Methoxypropylamin 28,8 g Butoxypropylamin eingesetzt werden. Das Reaktionsprodukt wird in einer Ausbeute von 22,6 g (89,5%) erhalten.

| | | | | |
|---|---|---|---|---|
| Analyse: | ber. | C 73,7% | gef. | C 73,3% |
| | | H 4,5% | | H 4,5% |
| | | N 2,8% | | N 2,7% |

Beispiel 10

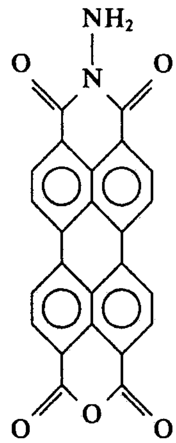

Ein Gemisch aus 300 ml Wasser, 13,8 g Hydrazinhydrat (80%ig) und 22,4 g Monokaliumsalz des Perylentetracarbonsäuremonoanhydrids wird auf 90°C erwärmt und 3 Stunden bei dieser Temperatur gehalten. Anschließend wird mit 500 ml Wasser verdünnt und heiß geklärt. Das aus dem Filtrat mit Säure gefällte und wie üblich isolierte Reaktionsprodukt wird durch Behandlung mit 400 ml heißer 5%iger Kalilauge in das Dikaliumsalz überführt. Nach üblicher Aufarbeitung werden 18,2 g (89,7%) Perylentetracarbonsäuremonoanhydridmono-N-aminoimid erhalten.

| | | | | | | |
|---|---|---|---|---|---|---|
| Analyse: | ber. | C | 70,9% | gef. | C | 70,4% |
| | | H | 2,5% | | H | 2,4% |
| | | N | 6,9% | | N | 6,9% |

Beispiel 11

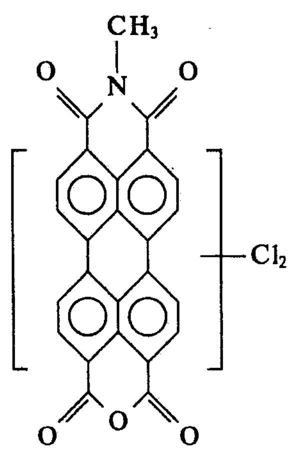

Gemäß Beispiel 6 werden 20,7 g Monokaliumsalz des Dichlorperylentetracarbonsäuremonoanhydrids, hergestellt gemäß Beispiel 10 der Patentanmeldung 81 101 315.0 aus Dichlorperylentetracarbonsäuredianhydrid, in 250 ml Wasser mit 10,8 g 45,4%iger Methylaminlösung zum entsprechenden Monoanhydridmonomethylimid umgesetzt.

Ausbeute: 10,4 g (54,8%).

Analyse: ber. N 3,0%, gef. N 3,1%.

Beispiel 12

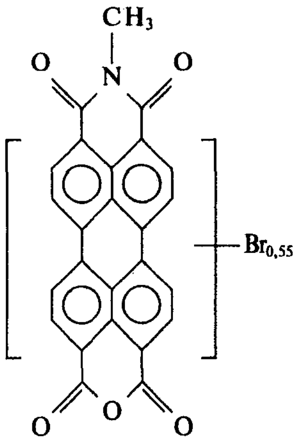

21,2 g Monokaliumsalz des Bromperylentetracarbonsäuremonoanhydrids hergestellt gemäß Beispiel 9 der Patentanmeldung 81 101 315.0 werden in 250 ml Wasser suspendiert und 9,8 g Methylaminlösung (45,4%ig) zugegeben. Man läßt 2 Stunden bei 20—25°C nachrühren und anschließend 28 g 50%ige Kalilauge zulaufen. Die Mischung wird 1 Stunde bei 90°C gehalten, dann auf 20°C abgekühlt und filtriert. Aus dem so erhaltenen Dikaliumsalz wird gemäß Beispiel 6 das Reaktionsprodukt in einer Ausbeute von 16,5 g (92%) isoliert.

| Analyse: | ber. | | gef. | |
|---|---|---|---|---|
| | | Br 9,8% | | Br 10,3% |
| | | N 3,1% | | N 3,3% |

Die folgende Tabelle enthält weitere Perylen-3,4,9,10-tetracarbonsäuremonoanhydridmonoimide, die entsprechend den in den obigen Beispielen beschriebenen Arbeitsweisen erhalten werden:

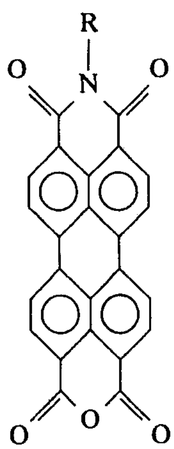

| Beispiel | R | Analyse ber. | gef. |
|---|---|---|---|
| 13 | $—(CH_2)_7CH_3$ | 2,8% N | 2,9% N |
| 14 | $—(CH_2)_3OCH_2CH(CH_2)_3CH_3$<br>$\vert$<br>$C_2H_5$ | 2,5% N | 2,7% N |
| 15 | $—CH_2CHCH_3$<br>$\vert$<br>$OH$ | 3,1% N | 3,0% N |

Fortsetzung

| Beispiel | R | Analyse ber. | gef. |
|---|---|---|---|
| 16 | $-(CH_2)_2N(C_2H_5)_2$ | 5,7% N | 5,5% N |
| 17 | 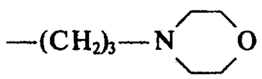 | 5,4% N | 5,1% N |
| 18 | $-OH$ | 3,4% N | 3,5% N |
| 19 | 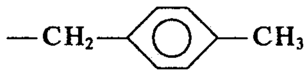 | 2,8% N | |
| 20 | 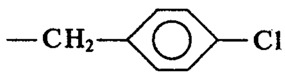 | 6,9% Cl | |

**Patentansprüche**

1. Verbindungen der allgemeinen Formel 1

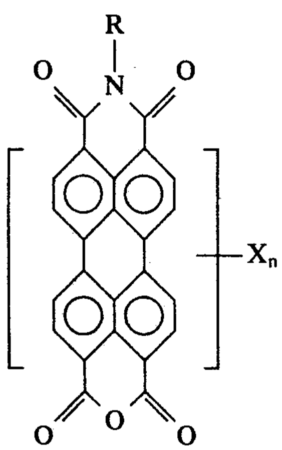

(1)

in der

R Wasserstoff,
Hydroxy,
Amino,
Cycloalkyl mit 4 bis 8 C-Atomen,
Alkyl mit 1 bis 8 C-Atomen, das substituiert sein kann durch
- Cycloalkyl mit 4 bis 8 C-Atomen,
- Phenyl, das seinerseits substituiert sein kann durch
  - Halogen,
  - niederes Alkyl,
  - niederes Alkoxy oder
  - niederes Alkoxycarbonyl,
- Cyan,
- Hydroxy,
- Carbamoyl,
- Acyl,
- niederes Dialkylamino,
- Morpholyl,
- Piperidyl oder
- Alkoxy mit 1 bis 8 C-Atomen, das seinerseits substituiert sein kann durch
  - Hydroxy,

niederes Alkoxy,
niederes Hydroxyalkyl,
Cyan,
Cycloalkyl mit 4 bis 8 C-Atomen oder
Phenyl,

X Chlor oder Brom und
n eine Zahl von 0 bis 4

bedeuten, wobei R nicht die Gruppe

$$—(CH_2)_y—H$$

in der y 0 bis 4 ist, bedeutet, wenn n Null ist.

2. Verfahren zur Herstellung von Perylen-3,4,9,10-tetracarbonsäuremonoanhydridmonoimiden der Formel (1), in der R für Wasserstoff, Hydroxy, Amino oder eine gegebenenfalls substituierte Alkylgruppe steht, X Chlor oder Brom und n eine Zahl von 0—4 bedeutet, dadurch gekennzeichnet, daß man Perylen-3,4,9,10-tetracarbonsäuremonoanhydridmonoalkalisalze der Formel 2

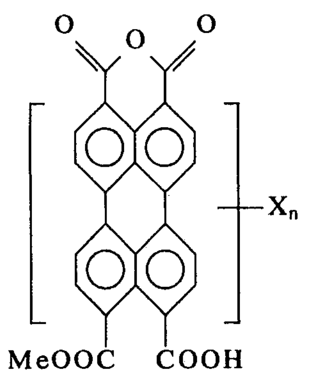

(2)

in der Me für Natrium oder Kalium steht und X und n die vorstehend genannte Bedeutung haben, mit Verbindungen der Formel 3

$$R—NH_2 \qquad (3)$$

worin R die vorstehend genannte Bedeutung hat, bei 0—130°C umsetzt.

3. Verfahren nach Anspruch 2, dadurch gekennzeichnet, daß Me Kalium bedeutet und n = 0 ist.

4. Verfahren nach Anspruch 2 und 3, dadurch gekennzeichnet, daß man die Umsetzung bei 0—95°C durchführt.

5. Verwendung der gemäß Anspruch 2 bis 4 erhältlichen Verbindungen gemäß Anspruch 1 als Zwischenprodukte zur Herstellung von asymmetrisch N-substituierten Perylen-3,4,9,10-tetracarbonsäurediimiden.

**Claims**

1. Compounds of the general formula 1

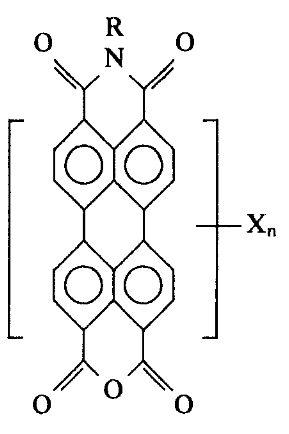

(1)

in which

R is hydrogen,
- hydroxy,
- amino,
- cycloalkyl with 4 to 8 C-atoms,
- alkyl of 1 to 8 carbon atoms which may be substituted by
  - cycloalkyl with 4 to 8 carbon atoms,
  - phenyl which may be substituted by
    - halogen,
    - lower alkyl,
    - lower alkoxy or
    - lower alkoxycarbonyl,
  - cyano,
  - hydroxy,
  - carbamoyl,
  - acyl,
  - lower dialkylamino,
  - morpholyl,
  - piperidyl, or
  - alkoxy of 1 to 8 carbon atoms which may be substituted by
    - hydroxy,
    - lower alkoxy,
    - lower hydroxyalkyl,
    - cyano,
    - cycloalkyl with 4 to 8 carbon atoms or
    - phenyl,

X is chlorine or bromine and
n is a number from 0 to 4,

and in which R is not the group

$$—(CH_2)_y—H$$

in which y is a number from 0 to 4, if n is zero.

2. A process for the preparation of perylene-3,4,9,10-tetracarboxylic acid monoanhydride monoimides of the formula (1) in which R is hydrogen, hydroxy, amino or an optionally substituted alkyl group, X is chlorine or bromine and n is a number from 0 to 4, which comprises reacting a monoalkali metal salt of perylene-3,4,9,10-tetracarboxylic acid monoanhydrides of the formula 2

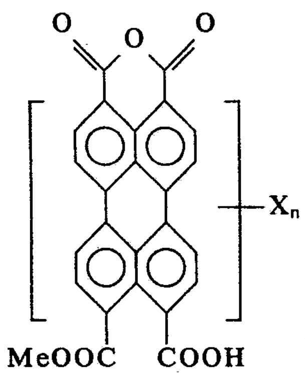

(2)

in which Me is sodium or potassium and X and n have the abovementioned meaning, with compounds of the formula 3

$$R—NH_2 \qquad (3)$$

in which R has the abovementioned meaning, at 0—130°C.

3. A process as claimed in claim 2, wherein Me is potassium and n is 0.

4. A process as claimed in claims 2 and 3, wherein the reaction is carried out at 0—95°C.

5. The use of the compounds obtained according to claims 2 to 4 according to claim 1 as intermediary products for the preparation of asymetrically N-substituted perylene-3,4,9,10-tetracarboxylic acid diimides.

**Revendications**

1. Composés répondant à la formule générale 1:

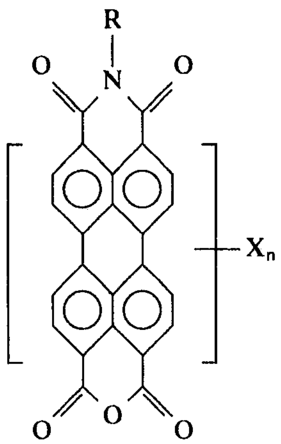

(1)

dans laquelle

R représente l'hydrogène, un hydroxy, un amino, un cycloalkyle contenant de 4 à 8 atomes de carbone, ou un alkyle contenant de 1 à 8 atomes de carbone, ce dernier pouvant porter: un cycloalkyle contenant de 4 à 8 atomes de carbone, un phényle éventuellement porteur d'un halogène, d'un alkyle inférieur, d'un alcoxy inférieur ou d'un alcoxycarbonyle inférieur, un cyano, un hydroxy, un carbamoyle, un acyle, un dialkylamino inférieur, un morpholyle, un pipéridyle ou un alcoxy contenant de 1 à 8 atomes de carbone, ce dernier pouvant porter: un hydroxy, un alcoxy inférieur, un hydroxyalkyle inférieur, un cyano, un cycloalkyle contenant de 4 à 8 atomes de carbone ou un phényle,
X représente un atome de chlore ou de brome et
n représente un nombre de 0 à 4,

avec la restriction que R ne peut, dans le cas où n est égal à 0, représenter un radical:

$$-(CH_2)_y-H$$

dans lequel y est un nombre de 0 à 4.

2. Procédé de préparation de mono-imides monoanhydrides d'acides pérylène-tétracarboxyliques-3,4,9,10 répondant à la formule 1 dans laquelle R représente l'hydrogène, un hydroxy, un amino ou un alkyle éventuellement substitué, X représente le chlore ou le brome et n représente un nombre de 0 à 4, procédé caractérisé en ce qu'on fait réagir, à une température de 0 à 130°C, un mono-sel de métal alcalin mono-anhydride d'acide pérylène-tétracarboxylique-3,4,9,10 répondant à la formule 2:

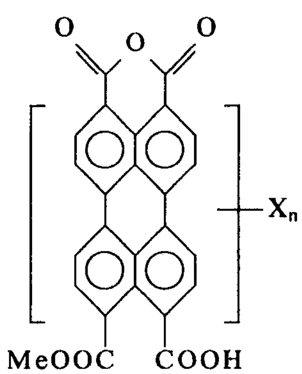

(2)

dans laquelle Me représente le sodium ou le potassium tandis que X et n ont les significations précédemment données, avec des composés répondant à la formule 3:

$$R-NH_2 \qquad (3)$$

dans laquelle R a la signification précédemment donnée.

3. Procédé selon la revendication 2 caractérisé en ce que Me représente le potassium et n est égal à 0.

4. Procédé suivant l'une des revendications 2 et 3, caractérisé en ce qu'on effectue la réaction à une température de 0 à 95°C.

5. Application des composés selon la revendication 1, qui peuvent être obtenus selon l'une quelconque des revendications 2 à 4, comme corps intermédiaires pour la préparation de di-imides d'acides pérylène-tétracarboxyliques-3,4,9,10 substitués aux atomes d'azote de façon asymétrique.